Europäisches Patentamt

European Patent Office

Office européen des brevets

(19)

(11) Veröffentlichungsnummer : **0 197 262
B1**

(12) **EUROPÄISCHE PATENTSCHRIFT**

(45) Veröffentlichungstag der Patentschrift :
**13.12.89**

(51) Int. Cl.⁴ : **A 61 F   2/38**

(21) Anmeldenummer : **86101824.0**

(22) Anmeldetag : **13.02.86**

(54) **Tibiateil einer Kniegelenk-Endoprothese.**

(30) Priorität : **01.03.85 DE 3507155**

(43) Veröffentlichungstag der Anmeldung :
**15.10.86 Patentblatt 86/42**

(45) Bekanntmachung des Hinweises auf die Patenterteilung : **13.12.89 Patentblatt 89/50**

(84) Benannte Vertragsstaaten :
**AT BE CH DE FR GB IT LI LU NL SE**

(56) Entgegenhaltungen :
**DE--A-- 2 400 834
DE--A-- 3 136 636**

(73) Patentinhaber : **S + G IMPLANTS GMBH
Grapengiesserstrasse 21
D-2400 Lübeck (DE)**

(72) Erfinder : **Grundei, Hans
Gärtnergasse 4
D-2400 Lübeck (DE)**
Erfinder : **Aigner, Reinhard, Dr.
Ismainger Strasse 22
D-8000 München 80 (DE)**

(74) Vertreter : **Wilcken, Thomas, Dipl.-Ing. et al
Musterbahn 1
D-2400 Lübeck (DE)**

## Beschreibung

Die Erfindung bezieht sich auf den Tibiateil einer Kniegelenk-Endoprothese als Ersatz für einen resezierten Tibiakopf, bestehend aus einem im Schienbein zu verankernden Schaft und einem oben das Tibia-Endoprothesen-Gelenkteil tragenden Überbrückungsteil, welches mit einem anschraubbaren Klemmelement für die Befestigung von natürlichen oder künstlichen Bändern (Sehnen) versehen ist.

Bei einer Resektion eines Tibiakopfes kommt eine Endoprothese als Ersatz zur Anwendung, die mit einem Schaft im Schienbein verankert wird. Durch die Resektion wird auch das natürliche, nach unten gerichtete Band (Sehne) der Patella durchtrennt, und es muß dann die Patella oder der verbleibende Teil des natürlichen Bandes mit einem oder zwei künstlichen Bändern verbunden werden, welche dann am Überbrückungsteil der Tibiakopf-Endoprothese zu befestigen sind. Dieses Befestigen erfolgte bisher durch Schellen, durch wickelbare Befestigungsdräthe oder dadurch, daß das natürliche oder künstliche Band mit einer anschraubbaren Klemmplatte gegen eine zweite. Platte festgehalten wurde, wie sich z. B. aus der DE-OS 24 00 834 ergibt. Dabei wurde das Band einmal oder mehrere Male scharf umgelenkt, so daß es hier durch das Festklemmen oder zumindest nach bestimmter Zeit beschädigt werden konnte und schließlich durchtrennt wurde. Die Klemmplatten wurden bei dieser Ausführung an den Knochen des Patienten angeschraubt.

Die Aufgabe der Erfindung besteht bei der Implantation des Tibiateiles einer Kniegelenk-Endoprothese darin, natürliche oder künstliche Bänder mit dem zu resizierenden Tibiakopf ersetzenden Tibiateil einer Endoprothese so verbinden zu können, daß die Bänder nicht über scharfe Kanten oder Löcher verlaufen müssen und dadurch beschädigt werden konnten und schließlich reißen.

Diese Aufgabe wird bei dem eingangs erwähnten Tibiateil einer Kniegelenk-Endoprothese dadurch gelöst, daß der Überbrückungsteil mit einer äußeren konkaven, abgerundete Kanten aufweisenden Mulde zur Aufnahme eines durch das konvexes Klemmelement in die konkave Mulde drückbaren Bandendes versehen ist. Vorteilhaft geht man dabei so vor, daß die konvexe Mulde an dem Unterende einer mit dem Überbrückungsteil fest verbundenen Lasche vorgesehen ist und daß diese Lasche oberhalb der Fläche der konkaven Mulde in eine nach außen vorspringende Bogenfläche übergeht.

Durch diese Lösung nach der Erfindung werden die festzuklemmenden Bänder auf einfache Weise sicher und dauerhaft ohne Beschädigungen fest mit dem Überbrückungsteil des Implantats verbunden. Durch diese Einklemmart ist es auch möglich, zunächst das eine oder beide künstlichen Bänder zwischen dem Klemmelement und dem Überbrückungsteil bzw. der mit dem Überbrückungsteil verbundenen Lasche hindurchzuführen und die Enden zum Spannen zu erfassen, bis die Patella ihre erforderliche Lage erreicht hat und richtig eingestellt ist, worauf das Klemmelement fest angeschraubt werden kann und dadurch das Band oder die Bänder eindeutig festklemmt.

Die Erfindung wird nachstehend anhand der Zeichnung beschrieben. Es zeigen :

Figur 1 eine Seitenansicht des Tibiateiles einer Kniegelenk-Endoprothese,

Figur 2 eine Vorderansicht des Tibiateiles nach Figur 1.

Der Ersatz für einen resezierten Tibiakopf einer Kniegelenk-Endoprothese besteht in bekannter Weise aus einem metallischen Überbrückungsteil 1, der mit einem Schaft 2 im Schienbein zu verankern ist und oben mit dem Gelenkteil 3 versehen ist, auf dessen beidseitig eines Steges 4 befindlichen Gleitflächen sich die Kufen des zugehörigen bekannten, nicht dargestellten Femurgelenkteiles abstützen.

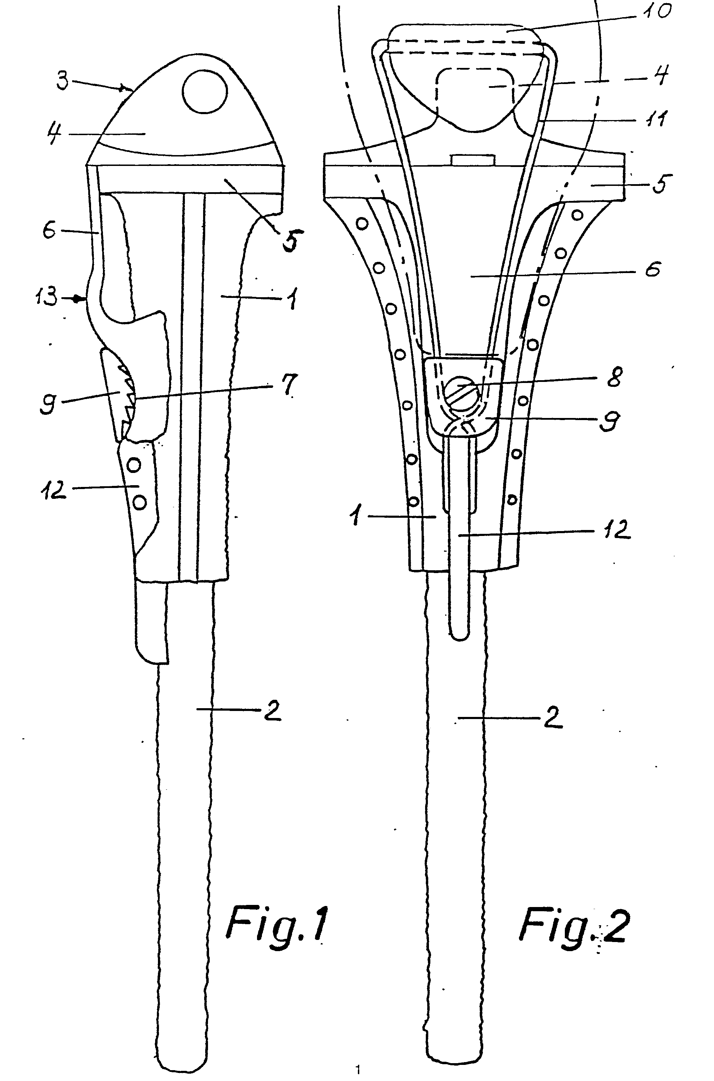

Der Überbrückungsteil 1 ist im Bereich eines oberen Kragens 5 auf der Vorderseite mit einer nach unten gerichteten Lasche 6 versehen, die zusammen mit einer anschraubbaren Scheibe 9 ein Klemmelement bildet. Die Lasche 6 ist am Unterende mit einer konkaven, vorteilhaft zylindrischen Anlagefläche 7 versehen, der sich die konvexe Rückseite der durch eine Schraube 8 mit der Lasche 6 zu verbindenden Scheibe 9 anpaßt. Die Oberfläche der Anlagefläche 7 ist vorteilhaft fein angerauht und die gegenüberliegende Rückseite der Scheibe 9 profiliert, z. B. sägezahnförmig profiliert, wie es in Figur 1 angedeutet ist.

Für das Implantieren des Tibiaprothesenteiles wird die in Figur 2 mit 10 bezeichnete Kniescheibe oder das gestrichelt angedeutete natürliche Einspannband der Kniescheibe mit einem Band 11 aus einem geeigneten Kunststoff verbunden, das nach unten zwischen der Klemmscheibe 9 und der Lasche 6 hindurchgeführt und hinter der Schraube 8 unter Kreuzung verlegt wird. Damit hat der Arzt es in der Hand, die Kniescheibe 10 durch Erfassen und ziehen des Bandes 11 die genaue erforderliche Lage der Kniescheibe 10 einzustellen und dann die Schraube 8 anzuziehen, womit das Band 11 zwischen der Scheibe 9 und der Lasche 6 festgeklemmt wird. Dabei unterstützt die Profilierung der Scheibenrückseite und die Aufrauhung der konkaven Anlagefläche der Lasche das Festklemmen des Bandes 11. Die nach unten gerichteten freien Enden des Bandes 11 werden vorteilhaft an einer mit Bohrungen versehenen Schiene 12 unterhalb der Lasche 6 z. B. durch Annähen festgelegt.

Wie sich aus Figur 1 ergibt, laufen die zwischen der Mulde 7 und dem Klemmelement 9 hindurchgelegten Bänder 11 nicht über scharfe Kanten, und weiter ist die Lasche 6 im Anschluß an die konkave Mulde 7 mit einem oben anschließenden, nach außen vorspringenden Bogen 13 versehen, der glatt in die Muldenfläche 7 übergeht. Dieser Bogen hat auch den Vorteil, daß das Band bei Beugebewegungen des Beines keinen allzu großen Druck auf die Gelenkteile der Prothese ausüben kann.

## Patentansprüche

1. Tibiateil einer Kniegelenk-Endoprothese als Ersatz für einen resezierten Tibiakopf, bestehend aus einem Schaft (2) mit einem oben das Tibiagelenkteil (3) tragenden Überbrückungsteil (1), welches mit einem anschraubbaren Klemmelement (9) für die Befestigung von natürlichen oder künstlichen Bänderenden versehen ist, dadurch gekennzeichnet, daß der Überbrückungsteil (1) mit einer äußeren, konkaven, abgerundete Kanten aufweisenden Mulde (7) zur Aufnahme eines durch das konvexe Klemmelement (9) in die konkave Mulde drückbaren Bandendes versehen ist.

2. Tibiateil nach Anspruch 1, dadurch gekennzeichnet, daß die konkave Mulde (7) an dem Unterende einer am Überbrückungsteil (1) befestigten Lasche (6) vorgesehen ist.

3. Tibiateil nach Anspruch 1 oder 2, dadurch gekennzeichnet, daß die Lasche (6) oberhalb der Fläche der konkaven Mulde (7) in eine nach außen vorspringende Bogenfläche (13) übergeht.

4. Tibiateil nach einem der Ansprüche 1 bis 3, dadurch gekennzeichnet, daß die Lasche (6) unterhalb der konkaven Mulde (7) mit einem durchlochten Steg (12) zum Annähen von Bandenden versehen ist.

5. Tibiateil nach einem der Ansprüche 1 bis 4, dadurch gekennzeichnet, daß die einander zugekehrten Flächen der konkaven Mulde (7) und des Klemmelementes (9) profiliert oder aufgerauht sind.

## Claims

1. A tibia part of a knee joint endoprosthesis as a replacement for a resectioned tibia head, comprising a shaft (2) having a bridging part (1) carrying at the top the tibia joint part (3) and provided with a clamping element (9) screwable thereto for the fastening of natural or artificial strap ends, characterised in that the bridging part (1) is provided with an outer, concave depression (7) having rounded edges for reception of a strap end arranged to be pressed into the concave depression by the convex clamping element (9).

2. A tibia part according to claim 1, characterised in that the concave depression (7) is provided at the lower end of a flap (6) fastened to the bridging part (1).

3. A tibia part according to claim 1 or 2, characterised in that the flap (6) merges into an outwardly protruding arcuate surface (13) above the area of the concave depression (7).

4. A tibia part according to one of the claims 1 to 3, characterised in that the flap (6) is provided below the concave depression (7) with a perforated web (12) fur the sewing-on of strap ends.

5. A tibia part according to one of the claims 1 to 4, characterised in that the surfaces of the concave depression (7) and of the clamping element (9) facing each other are profiled or roughened.

## Revendications

1. Partie tibiale d'une endoprothèse de l'articulation du genou comme pièce de substitution à une tête tibiale réséquée, constituée d'une tige (2) ayant une pièce de pontage (1) portant à sa partie supérieure la pièce tibiale d'articulation (3), et munie d'un élément de serrage (9) vissable pour la fixation d'extrémités de rubans naturels ou synthétiques, caractérisée en ce que la pièce de pontage (1) présente une cavité (7) externe et concave, à bords arrondis, pour le logement d'une extrémité de ruban destinée à être comprimée dans la cavité concave par l'élément de serrage (9) convexe.

2. Partie tibiale selon la revendication 1, caractérisée en ce que la cavité concave (7) est prévue à l'extrémité inférieure d'une attache (6) fixée à la pièce de pontage (1).

3. Partie tibiale selon la revendication 1 ou 2, caractérisée en ce que l'attache (6) se prolonge, au-dessus de la surface de la cavité concave (7), par une surface cintrée (13) en saillie vers l'extérieur.

4. Partie tibiale selon l'une des revendications 1 à 3, caractérisée en ce que l'attache (6) présente, sous la cavité concave (7), une âme percée pour la fixation par couture d'extrémités de rubans.

5. Partie tibiale selon l'une des revendications 1 à 4, caractérisée en ce que les faces tournées l'une vers l'autre de la cavité concave (7) et de l'élément de serrage (9) sont profilées òu non lisses.

EP 0 197 262 B1

*Fig.1*

*Fig.2*